# EUROPÄISCHE PATENTANMELDUNG

(11) **EP 4 163 359 A1**
(43) Veröffentlichungstag der Anmeldung: **12.04.2023**
(21) Anmeldenummer: 22200444.2
(22) Anmeldetag: 07.10.2022
(51) Int. Cl.: C12M 1/107, C12M 1/33, C12M 1/00

(54) **REGENERATIVES SPEICHERKRAFTWERK MIT RÜCKFÜHRUNG VON BIOMATERIAL**

(30) Priorität: 11.10.2021 DE 102021126275
(71) Anmelder: Maier, Clemens, 88316 Isny (DE); Maier, Gregor, 88316 Isny (DE)
(72) Erfinder: Maier, Clemens, 88316 Isny (DE); Maier, Gregor, 88316 Isny (DE)
(74) Vertreter: Otten, Roth, Dobler & Partner mbB Patentanwälte

(57) **Zusammenfassung**

Vorgeschlagen wird ein hocheffizientes Verfahren zum Betrieb eines Biomassekraftwerks mit einem Fermenter zum Zersetzen von Biomasse, einem Verdampfer zum Eindampfen von Biomasse und einem Separator zur Trennung von flüssiger und fester Biomasse, wobei in einem ersten Schritt des Verfahrens aus festen und flüssigen Bestandteilen bestehende Biomasse aus wenigstens einer Anmischgrube und/oder einer Vorgrube und/oder Flüssigfütterung in einen Fermenter gepumpt wird und in einem zweiten Schritt die Biomasse weiter in einen mechanischen Separator gepumpt und dort in eine feste Biomasse und eine flüssige Biomasse getrennt wird, wobei die flüssige Biomasse in einem dem Separator nachgeschalteten Verdampfer eingedampft wird. Hierzu wird das feste Biomaterial nach Abtrennung von flüssiger Biomasse im Separator in wenigstens eine Anmischgrube und/oder den Fermenter rückgeführt.

## Beschreibung

Die Erfindung betrifft ein Verfahren zum Betrieb eines Biomassekraftwerks mit wenigstens einer Anmischgrube zum Vermengen von fester und flüssiger Biomasse und/oder einer Vorgrube, die als Vorlage dient, gemäß dem Oberbegriff des Anspruchs 1.

### Stand der Technik:

Bei bisherigen Biomassekraftwerken wird Biomasse in Vorgruben und/oder Anmischgruben für die eigentliche Vergährung im Fermenter vorbereitet oder direkt über Feststoffeintragsystem direkt dem Fermenter zugeführt. Während der Vergährung zersetzt sich die Biomasse teilweise, wodurch Energie gewonnen wird. Nach einer gewissen Gährzeit wird die Biomasse aus dem Fermenter durch neue Biomasse aus den Vorgruben bzw. Anmischgruben ersetzt und weiter verwendet. Dabei wird das Substrat mehrmals am Tag in Intervallen getauscht oder auch nur in Teilmengen, die zur Fütterung benötigt wird, entnommen. Eine solche Ausführung ist beispielsweise aus der WO 2020/057697 A1 bekannt.

### Aufgabe und Vorteile der Erfindung:

Aufgabe der Erfindung ist es demgegenüber, ein Verfahren zum Betrieb eines Biomassekraftwerks mit wenigstens einer Anmischgrube zum Vermengen von fester und flüssiger Biomasse und/oder einer Vorgrube die als Vorlage dient, vorzuschlagen, das die Effizienz und Energiegewinnung des Biomassekraftwerks verbessert.

Diese Aufgabe wird, ausgehend von einem Verfahren der einleitend genannten Art, durch die Merkmale des Anspruchs 1 gelöst. Durch die in den Unteransprüchen genannten Maßnahmen sind vorteilhafte Ausführungen und Weiterbildungen der Erfindung möglich.

Dementsprechend zeichnet sich ein erfindungsgemäßes Verfahren dadurch aus, dass das feste Biomaterial nach Abtrennung von flüssiger Biomasse im Separator in eine der wenigstens einen Anmischgruben und/oder den Fermenter rückgeführt wird.

Bei der Fermentierung wird die Biomasse nach und nach zersetzt, wodurch Energie freigesetzt wird. Da der flüssige Anteil bereits weitestgehend zersetzt und energetisch auf niedrigem Level ist, kann aus diesem kaum mehr Energie gewonnen werden.

Der Separator trennt die festen Bestandteile, welche durch ihre Größe noch ein zur Energiegewinnung nutzbares Energielevel aufweisen, vom flüssigen Anteil, sodass der feste Anteil in eine oder mehrere Anmischgruben und/oder den Fermenter rückgeführt wird. In der Anmischgrube werden die festen Bestandteile mit frischer Biomasse mit einem Rührwerk durchmischt, sodass sich die für den Gährungsprozess nötigen Bakterienkulturen in der Biomasse vermehren und gleichmäßig verteilen.

Durch die Rückführung der noch festen Bestandteile aus dem Separator in die Anmischgrube bzw. den Fermenter kann dieses Material weiter zersetzt werden. Statt das Material halbzersetzt zu entsorgen, wird es erneut zur Energiegewinnung genutzt. Als Konsequenz wird daher bei gleicher Menge Ausgangsmaterial zum einen weniger Abfallprodukt erzeugt und zweitens mehr Energie gewonnen. Gleichzeitig muss das Biomassenkraftwerk, insbesondere hinsichtlich der Teilanlagen Fermenter und Anmischgruben, nicht größer gebaut werden als bei bisher genutzten Anlagen ohne Rückführung. Das bedeutet, dass bereits beim Bau des Biomassenkraftwerks gegebenenfalls auf bauliche Anlagen wie Rührwerke oder andere Ausbauten eingespart werden können.

In einer bevorzugten Ausbildungsvariante wird die Biomasse zwischen Fermenter und Separator mittels eines Nasszerkleinerers zerkleinert bzw. aufgefasert. Durch diese Zerkleinerung der anfermentierten Fasern kann die Trennung von flüssigem Material und festem Material im Separator einfacher ausgeführt werden, da so weniger Kapazitäten für die Flüssigkeit innerhalb oder zwischen den festen Bestandteilen bleiben.

Bevorzugterweise wird die eingedampfte flüssige Biomasse in einem Gärproduktlagerbehälter zur Nachgärung und/oder Abfüllung und/oder das beim Eindampfen freigesetzte Wasser in einem Wasserspeicher zwischengelagert. Während die eingedampfte flüssige Biomasse zur Entsorgung abgeholt wird, kann das entstandene Wasser weiter genutzt werden, wie beispielsweise zur Reinigung der Anlage oder auch anderweitig wie zur Bewässerung von Feldern.

In einer möglichen Ausführung wird das beim Eindampfen entstehende Wasser in die Vorgrube und/oder die Anmischgrube und/oder den Fermenter rückgeführt. Durch die teils wiederholte Rückführung des festen Bestandteils der fermentierten Biomasse vom Separator zurück in Anmischgrube oder Fermenter, wird die Biomassenmischung dort eingedickt. Üblicherweise ist das Eingangssubstrat (z.B. Grassilage oder dergl.) entsprechend kurz geschnitten, sodass eine Eindickung kein Problem darstellt, obwohl sich die Viskosität im Substrat erhöht. Gleichzeitig erhöht sich auch die Homogenität des Fermenterinhalts bei einer höheren Viskosität. Bei extremer Eindickung, was bei speziellen Substraten vorkommen kann, kann dann zur Vorbeugung von Verstopfungen Wasser zur Verdünnung der Mischung hinzugefügt werden. Hierfür kann auch aus Kostengründen und zur Einsparung von Ressourcen das bereits im System befindliche Wasser, das beim Eindampfen der flüssigen Biomasse entsteht, genutzt werden. Gleichzeitig kann insbesondere bei der Vergärung von stickstoffreichem Material, wie beispielsweise Hühnertrockenkot, durch die Zugabe von Wasser auch der Stickstoffgehalt sowie der pH-Wert der Biomassenmischung reguliert werden, was beides Einfluss auf den Ablauf des Gärprozesses hat, sodass sich hieraus eine Anwendungsmöglichkeit ergibt. Beispielsweise kann ein zu hoher Ammoniumanteil im Fermenterinhalt den Gärprozess und damit die Methanbildung vollständig zum Erliegen bringen. Durch Einbringen von Wasser in die Mischung kann der relative Ammoniumanteil reduziert werden.

Es ist von Vorteil, wenn die beim Verdampfer rückgewonnene Wärmeenergie dazu genutzt wird, Biomasse in der wenigstens einen Anmischgrube und/oder dem Fermenter zu erwärmen. Die Temperatur bei normalen Bioprozessen lässt die Biomasse auf etwa 45°C ansteigen. Durch die Zuführung von Wärmeenergie in die Anmischgrube bzw. den Fermenter kann die Temperatur auf etwa 60°C erhöht werden, wodurch die Fermentierung zügiger stattfinden kann. So kann im Prozess der Fermentierung der Biomasse Zeit eingespart werden, sodass in gleicher Zeit mehr Masse fermentiert und dadurch mehr Energie gewonnen werden kann.

Idealerweise werden wenigstens zwei dem Fermenter vorgeschalteten Anmischgruben verwendet und diese zyklisch versetzt zueinander befüllt und entleert. Das heißt, während Biomasse aus der ersten Anmischgrube entnommen wird um den Fermenter zu speisen, wird die zweite Anmischgrube mit neuer Biomasse aufgefüllt. In der Zeit, in der die erste Anmischgrube wieder beschickt wird, kann die zweite Anmischgrube zur Befüllung des Fermenters herangezogen werden. Dadurch kann garantiert werden, dass stets ausreichend vorbereitete Biomasse für die Fermentierung im Fermenter zur Verfügung steht, um diese zur Steigerung der Effizienz durchgehend ablaufen lassen zu können. Im Gegensatz zu einem Biomassekraftwerk mit einer einzelnen Anmischgrube, bei der nach einer Entnahme der Biomasse die Anmischgrube diese erst wieder befüllt und aufbereitet werden muss, bevor eine weitere Entnahme erfolgen kann, kann so eine wesentliche Zeiteinsparung erzielt werden.

### Beschreibung eines Ausführungsbeispiels:

Ein Ausführungsbeispiel der Erfindung ist in der Zeichnung dargestellt und wird anhand der Figuren nachfolgend näher erläutert.

Im Einzelnen zeigt:
- Figur 1: einen schematischen Aufbau eines Biomassekraftwerks gemäß der Erfindung und

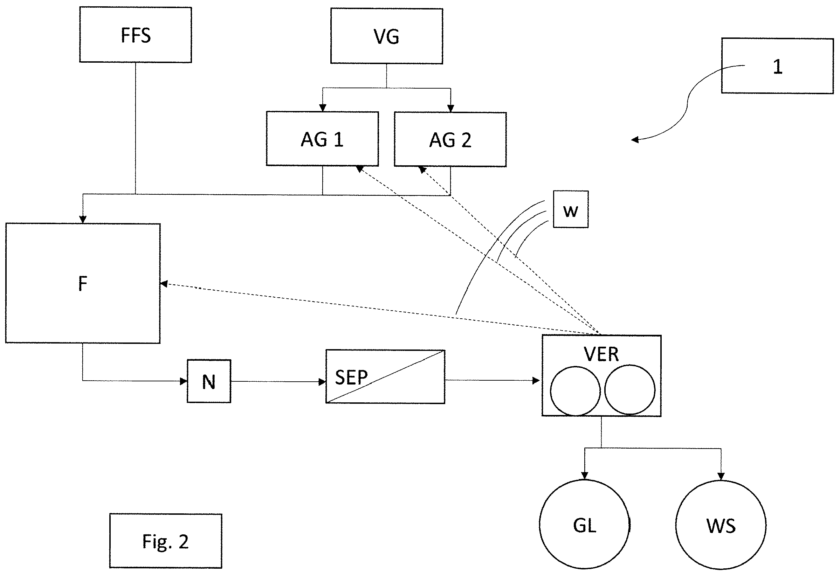

- Figur 2: einen schematischen Aufbau eines Biomassekraftwerks gemäß einer Weiterbildung der Erfindung

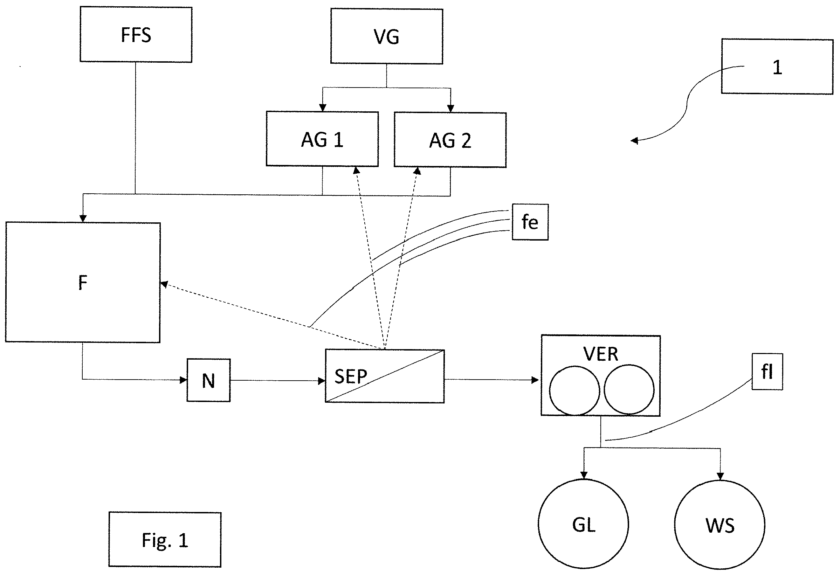

In Figur 1 ist ein Biomassekraftwerk 1 schematisch in seinen einzelnen Abschnitten dargestellt.

In einem ersten Schritt des Verfahrens wird aus festen und flüssigen Bestandteilen bestehende Biomasse aus einer Vorgrube VG, die als Vorlage dient, wenigstens eine Anmischgrube AG 1, AG 2 zum Vermengen befüllt, wobei die Biomasse anschließend zur Fermentierung und daraus resultierender Energiegewinnung in einen Fermenter F geleitet wird. Denkbar ist auch, dass der Fermenter F direkt aus der Vorgrube VG oder mittels eines Flüssigfütterungssystems FFS befüllt wird.

In dem Fermenter F wird die Biomasse zersetzt, woraus Energie gewonnen wird. Die Biomasse innerhalb des Fermenters F wird diskontinuierlich durch neue Biomasse aus der Vorgrube VG bzw. den Anmischgruben AG 1, AG 2 ersetzt.

Die fermentierte Biomasse wird in einem zweiten Schritt über ein Rohrsystem zu einem Separator SEP geleitet. Auf dem Weg dorthin ist denkbar, dass ein Nasszerkleinerer N die Biomasse weiter zerkleinert bzw. auffasert. Im Separator SEP wird die Biomasse in feste Biomasse fe und flüssige Biomasse fl getrennt, wobei die flüssige Biomasse fl in einem dem Separator SEP nachgeschalteten Verdampfer VER eingedampft wird.

Während die eingedampfte flüssige Biomasse fl in einem Gärproduktlagerbehälter GL zur Nachgärung und späteren Weitzerverwendung gelagert wird, kann das Wasser in einem Wasserspeicher WS für verschiedene weitere Verwendungen zwischengelagert werden.

Das feste Biomaterial fe, das zur besseren Transportfähigkeit noch immer einen gewissen Flüssigkeitsanteil aufweist, wird nach Abtrennung der flüssigen Biomasse fl im Separator SEP direkt in den Fermenter F bzw. abwechselnd in eine der Anmischgruben AG 1, AG 2 rückgeführt und mit zusätzlicher frischer Biomasse vermengt. So kann gewährleistet werden, dass das Energiepotential, das in den noch unvollständig zersetzten Fasern der Biomasse weiter ausgeschöpft wird.

In einer vorteilhaften Weiterbildung der Erfindung wird die Wärmeenergie w, welche beim Eindampfen des flüssigen Anteils fl der Biomasse im Verdampfer VER freigesetzt wird, wenigstens teilweise dem Fermenter F bzw. den Anmischgruben AG 1, AG 2 zugeführt. Die dort befindliche Biomasse wird so erwärmt, sodass die biochemische Reaktion der Fermentierung begünstigt wird und schneller abläuft. So kann in dem Biomassekraftwerk 1 in gleicher Zeit mehr Biomasse zersetzt und damit auch mehr Energie gewonnen werden.

### Bezugszeichenliste:

- 1: Biomassekraftwerks
- VG: Vorgrube
- AG 1: erste Anmischgrube
- AG 2: zweite Anmischgrube
- F: Fermenter
- N: Nasszerkleinerer
- SEP: Separator
- VER: Verdampfer
- GL: Gärproduktlagerbehälter
- WS: Wasserspeicher
- FFS: Flüssigfütterungssystem

- fe: feste Biomasse
- fl: flüssige Biomasse
- w: Wärmeenergie

## Patentansprüche

1. Verfahren zum Betrieb eines Biomassekraftwerks (1) mit wenigstens einer Anmischgrube (AG 1, AG 2) zum Vermengen von fester (fe) und flüssiger Biomasse (fl) und/oder einer Vorgrube (VG) die als Vorlage dient und/oder einem Flüssigfütterungssystem , einem Fermenter (F) zum Zersetzen von Biomasse, einem Verdampfer (VER) zum Eindampfen von Biomasse und einem Separator (SEP) zur Trennung von flüssiger (fl) und fester Biomasse (fe), wobei in einem ersten Schritt des Verfahrens aus festen (fe) und flüssigen Bestandteilen (fl) bestehende Biomasse aus wenigstens einer Anmischgrube (AG) und/oder einer Vorgrube (VG) und/oder Flüssigfütterung (FFS) in einen Fermenter (F) gepumpt wird und in einem zweiten Schritt die Biomasse weiter in einen mechanischen Separator (SEP) gepumpt und dort in eine feste Biomasse (fe) und eine flüssige Biomasse (fl) getrennt wird, wobei die flüssige Biomasse (fl) in einem dem Separator (SEP) nachgeschalteten Verdampfer (VER) eingedampft wird, **dadurch gekennzeichnet, dass** das feste Biomaterial (fe) nach Abtrennung von flüssiger Biomasse (fl) im Separator (SEP) in eine der wenigstens einen Anmischgruben (AG 1, AG 2) und/oder den Fermenter (F) und/oder das Flüssigfütterungssystem (FFS) rückgeführt wird.

2. Verfahren für ein Biomassekraftwerk (1) nach Anspruch 1, **dadurch gekennzeichnet, dass** die Biomasse zwischen Fermenter und Separator mittels eines Nasszerkleinerers (N) zerkleinert bzw. aufgefasert wird.

3. Verfahren für ein Biomassekraftwerk (1) nach einem der vorgenannten Ansprüche, **dadurch gekennzeichnet, dass** die eingedampfte flüssige Biomasse (fl) in einem Gärproduktlagerbehälter (GL) zur Abfüllung und/oder das beim Eindampfen freigesetzte Wasser in einem Wasserspeicher (WS) zwischengelagert wird.

4. Verfahren für ein Biomassekraftwerk (1) nach einem der vorgenannten Ansprüche, **dadurch gekennzeichnet, dass** das beim Eindampfen entstehende Wasser in die Vorgrube (VG) und/oder die Anmischgrube (AG 1, AG 2) und/oder den Fermenter (F) rückgeführt wird.

5. Verfahren für ein Biomassekraftwerk (1) nach einem der vorgenannten Ansprüche, **dadurch gekennzeichnet, dass** beim Verdampfer rückgewonnene Wärmeenergie (w) dazu genutzt wird, Biomasse in der wenigstens einen Anmischgrube (AG 1, AG 2) und/oder dem Fermenter (F) zu erwärmen.

6. Verfahren für ein Biomassekraftwerk (1) nach einem der vorgenannten Ansprüche, **dadurch gekennzeichnet, dass** wenigstens zwei dem Fermenter (F) vorgeschalteten Anmischgruben (AG 1, AG 2) verwendet werden und diese zyklisch versetzt zueinander befüllt und entleert werden.

7. Verfahren für ein Biomassekraftwerk (1) nach einem der vorgenannten Ansprüche, **dadurch gekennzeichnet, dass** bei der Rückführung der festen Biomasse (fe) vom Separator (SEP) in die wenigstens eine Anmischgrube (AG 1, AG 2) und/oder den Fermenter (F) eine Ultraschall-Zerkleinerung stattfindet.

8. Verfahren für ein Biomassekraftwerk (1) nach einem der vorgenannten Ansprüche, **dadurch gekennzeichnet, dass** für das Verfahren zwischen Anmischgruben (AG 1, AG 2) und Gärproduktlagerbehälter (GL) und Wasserspeicher (WS) ein in sich geschlossenes System verwendet wird, in dem zumindest ein Teil der Biomasse oder verarbeiteten Biomasse ohne Entnahme oder Zuführung von Stoffen in einem Kreislaufsystem zwischen Anmischgruben (AG 1, AG 2) und Gärproduktlagerbehälter (GL) zirkulieren kann.

9. Verfahren für ein Biomassekraftwerk (1) nach einem der vorgenannten Ansprüche, **dadurch gekennzeichnet, dass** die wenigstens eine Anmischgrube (AG 1, AG 2) zusätzlich mit flüssiger Biomasse (fl) aus einer der Anmischgrube (AG 1, AG 2) vorgeschalteten Vorgrube (VG) befüllt wird, welche separat von außen mit flüssiger Biomasse, insbeondere Gülle und Oberflächenwasser beschickt wird.
